Europäisches Patentamt

European Patent Office

Office européen des brevets

(19)

(11) Publication number: **0 040 077**
**B1**

(12) **EUROPEAN PATENT SPECIFICATION**

(45) Date of publication of patent specification: **04.07.84**

(21) Application number: **81302073.2**

(22) Date of filing: **11.05.81**

(51) Int. Cl.³: **A 01 K 29/00,**
**A 61 D 13/00**

(54) Device for the temperature measurement of female mammals.

(30) Priority: **12.05.80 US 149250**
**06.10.80 US 194583**

(43) Date of publication of application:
**18.11.81 Bulletin 81/46**

(45) Publication of the grant of the patent:
**04.07.84 Bulletin 84/27**

(84) Designated Contracting States:
**CH DE FR IT LI**

(56) References cited:
**DD - A - 141 355**
**US - A - 2 509 825**
**US - A - 4 091 807**

(73) Proprietor: **NEW MEXICO STATE UNIVERSITY**
**FOUNDATION, INC.**
**Les Cruces New Mexico (US)**

(72) Inventor: **Zartman, David Lester**
**1803 Debra, Las Cruces**
**New Mexico (US)**

(74) Representative: **Hind, Raymond Stenton et al,**
**Mathisen, Macara & Co. Lyon House Lyon Road**
**Harrow, Middlesex, HA1 2ET (GB)**

Courier Press, Leamington Spa, England.

## Description

The present invention relates to the temperature measurement of female mammals.

Livestock breeders, especially cattlemen, find that one of their major concerns is that of being able to maximize the conception rate of their females.

Dairymen have known for years that cow's body temperature is capable of foretelling the onset of estrus as well as conditions of poor health, like, for example, mastitis and other fever-inducing ailments. They also knew that a cow's temperature varied greatly with ambient conditions and were by no means the same from one animal to the next even under identical conditions. It has therefore been recognized that a long term temperature history of a particular cow related to the change in ambient conditions would be very helpful in detecting the small abnormalities in the temperature profile for a given animal that are needed for a reliable prediction of estrus or a febrile illness. Hitherto, there has not been an effective way of obtaining this information.

Implantations of temperature probes in the ear canal or rectum have not proved effective in obtaining sufficiently accurate temperature readings necessary for the reliable prediction of estrus or ill health. It has been determined that only temperature measurements taken deep inside the body are sufficiently free of ambient climatic factors and other external influences to a point where the necessary degree of accuracy is attainable under herd management conditions. Also, as a practical matter, relatively long term implants are necessary since the herd owner cannot afford the labour cost of having to take the deep body temperature of each individual animal separately at least once a day and preferably at the same time.

The best possibility for a true deep body temperature reading essentially unaffected by external conditions appears to be the vaginal canal, despite tests in human subjects which have shown to the contrary. The difficulty with probes implanted into the vaginal canal is that muscular action tends to expel the probe relatively quickly.

U.S. Patent Specification No. 4,091,807 shows a device which is intended to be inserted into the vagina of a female animal and retained therein over a long period in order to enhance the quality and quantity of meat produced by the animal. This device comprises a plurality of stars or spiders the ends of which are bulbous and are directed towards the cervix. It is intended that the bulbous ends should become embedded in the vaginal wall in order to retain the device in the vagina. The stem of this device projects beyond the stars or spiders and is provided with an annular cervical ring which is intended to encircle the animal's projecting cervix. It has been found that this device, although suitable for its intended purpose, is not retained in the vagina for a sufficiently long period of time to enable the monitoring of deep body temperature over an extended period.

According to the invention, there is provided a device for intravaginal implantation in a mammalian female comprising an axially extending hub encircled by at least two rings of radially-extending springable spine-like fingers co-operating with said hub, to define a multi-pointed star, characterised in that a respective said star is located at each of the opposed extremities of the hub which is devoid of any projecting portion accessible to the vaginal wall musculature, and in that temperature sensing means are mounted on said hub in nested protective relation among the fingers.

The invention will now be described, by way of example only, with reference to the accompanying diagrammatic drawings, in which:—

Figure 1 is a diagram showing placement of a probe and an anchor therefor in the vaginal canal of the animal,

Figure 2 is a perspective view of the probe and anchor;

Figure 3 shows a modified form of anchor; and

Figures 4—9 are charts detailing the long term intravaginal temperature response of various species of female mammals.

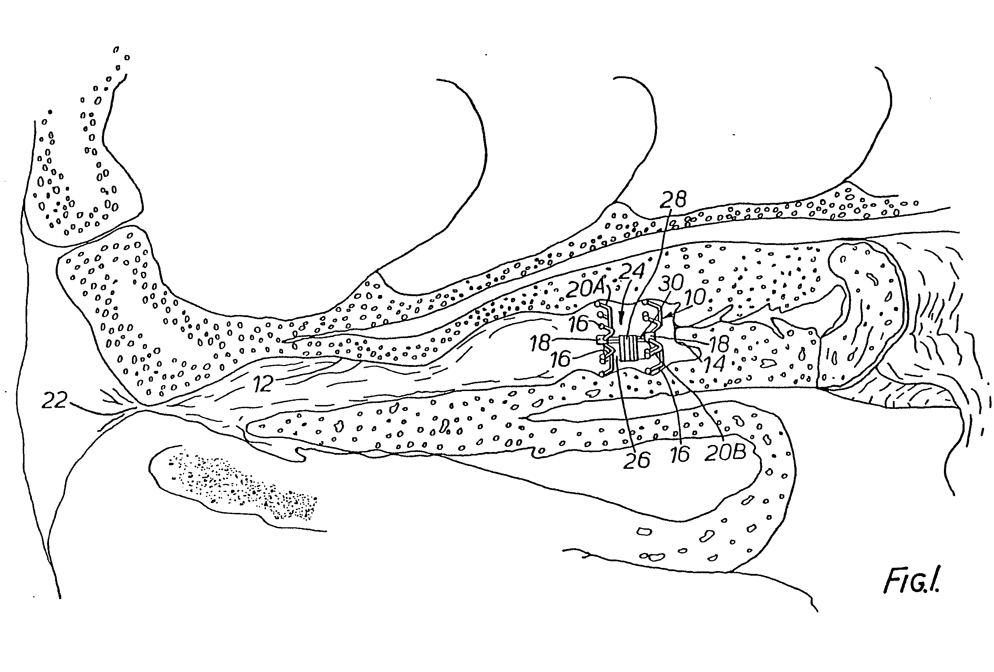

Referring initially to Figure 1, the temperature sensing probe used in the method is conventional and it consists of a battery powered transmitter containing a temperature-sensing thermistor which sends out a pulsed signal, the rapidity of which corresponds to the temperature of the transmitter and also the deep body temperature of the animal when implanted in her vaginal canal 12. The transmitter has been shown without detail but approximately its actual size in relation to a grown cow's vagina 12. The resulting signal is sensed at a remote location outside the animal's body. The location of the receiver is optional depending upon its sensitivity and the strength of the signal generated, some receivers being responsive to signals originating miles away from the transmitter. The selection of signal strength and receiver sensitivity is a matter of choice well within the skill of the art and will depend to a considerable degree upon several extraneous factors such as the size of the herd, the ability of the equipment to differentiate among the several animals, the degree of confinement of the herd, if any, and other similar parameters.

The signal that is received is recorded and analyzed during a preselected time period, say five minutes, at the same or approximately the same time each day. In bovine animals, for instance, it is a well-known fact that the lowest body temperatures of the day occur early in the morning between approximately 5:00 and 7:00 a.m. The baseline against which the spikes are most evident is likely to be established during such a time period; therefore, for cattle at least, the measurements are preferably made during this minimum baseline temperature interval.

For small confined herds, tunable receivers are unecessary provided the transmitted signal is relatively weak. In such a circumstance, the receiver is placed in reasonably close proximity to the particular transmitter whose temperature signal is to be decoded so as to screen out other extraneous signals. On the other hand, care should be taken to not excite the animal thus inducing a false spike.

The battery powered radio transmitter containing the temperature-sensitive thermistor is implanted at the mouth of the cervix 14, where, for purposes of making dependable and accurate predictions, it must stay for at least one complete estrus cycle and preferably fifty days or longer. Because of the stress-induced fluctuations in temperature, handling of the animal should be minimized; however, a 50 day test performed at the proper time will allow the animal to settle down while at the same time provide good baseline temperature data against which the spikes become easy to detect as will be apparent presently. Notwithstanding the long term implantation of the radio transmitter, it remains readily accessible to service, repair and change batteries, all without having to resort to surgical invasion of the animal or require the services of a veterinary surgeon.

The act of implantation is a simple one capable of being carried out by unskilled personnel using standard techniques and instruments like, for instance, a trochar tube and pushrod or plunger inside the latter. The problems do not arise in connection with placement of the probe but rather with how to keep it in place. The muscle contractions and relaxations of the vaginal walls are such that they quickly eject any foreign object like the probe. The problem is solved by an expandable anchoring device which is introduced into the vagina and which yields under the influence of the contracting vaginal muscles thus preventing them from getting a sufficient hold to eject either the anchor or the transmitter attached thereto back out through the vulva. One spider-like form of anchor which has been used with good results is shown in Figure 1 and identified by reference numeral 10.

The anchor 10 is implantable using well-known techniques within the vagina 12 of a mammalian female at the mouth of the cervix 14, the particular anatomy illustrated being that of a cow or heifer, although the animal could equally be a sow, a mare, or a ewe. The method of implantation is simply one of collapsing the radially-extending spider-like legs 16 emanating from the hub 18 of each star 20 preparatory to placing same in a speculum tube (not shown). Upon insertion of such a tube through the vulva opening 22 and ejecting the anchor from such a tube by means of a pushrod or the like (not shown) the placement of the unit is complete. Suitable precautions are, of course, taken to insure that no injury of the delicate tissue in the vaginal area takes place and that the entire procedure is carried out under antiseptic conditions.

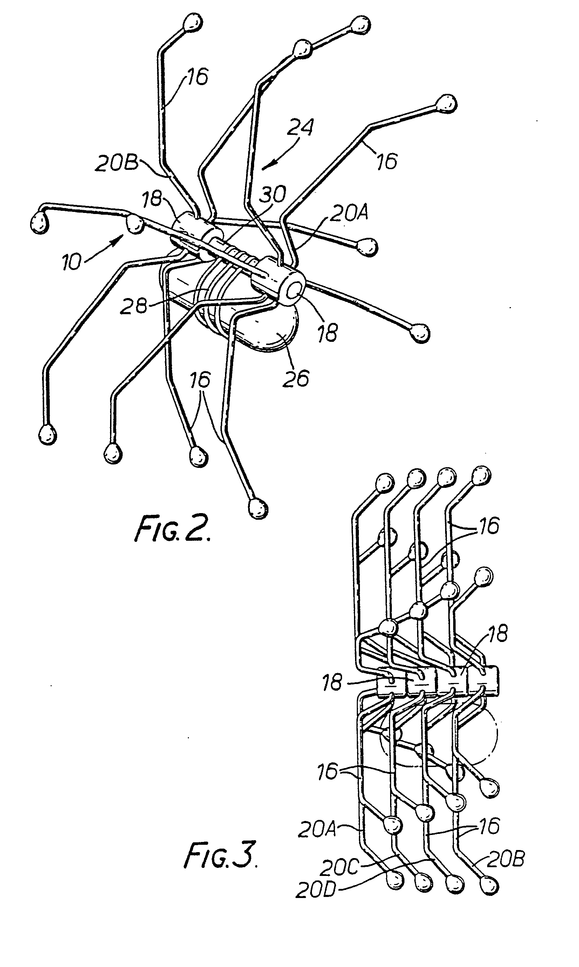

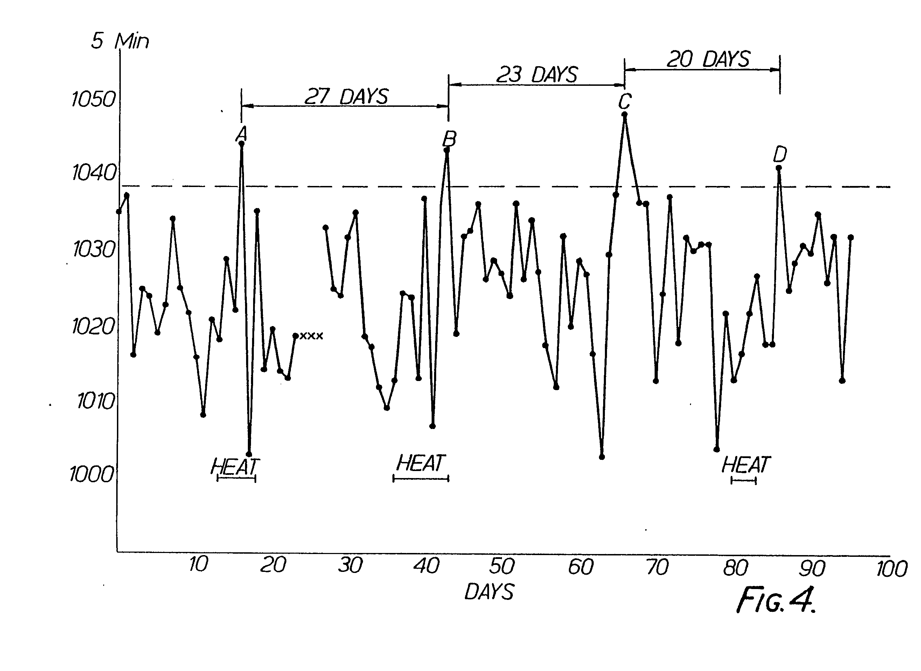

As soon as the anchor 10 leaves the trochar tube, its arms 16 spring open and assume the unfolded or expanded operative condition shown in all three figures of the drawings. As shown in Figures 1 and 2 in full lines and in phantom lines in Figure 3, the anchor 10 is part of an assembly 24 that includes, as discussed earlier, a conventional battery-powered radio transmitter 26 containing a temperature sensing thermistor which sends out a pulsed signal and some means 28 for fastening the latter to the hubs 18 intermediate the stars 20 on opposite ends thereof. The speculum tube must be sized to accommodate the entire assembly while, at the same time, remaining small enough to pass easily through the vulva opening.

Figures 1 and 2 to which detailed reference will next be made both show the 2-star version of the anchor in which identical stars 20 are spaced apart axially about 1.9 cms. with an axially-extending stem member 30 being used to interconnect the hollow hubs 18 of the two stars. As illustrated in Figure 2, the fastening means 28 comprises a short length of sterile cord or suture material tying the transmitter to the exposed medial portion of the stem. The 2-star version of the anchor 10 has proven quite satisfactory for use in heifers and small cows.

The 4-star version of the anchor shown in Figure 3 comprises two additional stars 20C and 20D interposed between stars 20A and 20B found in both the 2-star and 4-star versions. For the majority of heifers and cows the 4-star version is entirely adequate and should result in essentially 100% retention over extended period of time, say 50 days and longer. In extreme cases such as an old heavy cow, the preferred anchor would be a 6-star version (not shown). a 2-star unit has proven quite adequate as an anchoring device for holding small objects in the vagina of cows, heifers and mares of reproductive age although, to be on the safe side, the 4-star version is preferable for use in the more mature females.

The anchor used is similar to a vaginal device which is used to stimulate growth and is described in U.S. Patent Specification 4,091,807. This device comprises a plurality of stars or spiders and a projecting cone and stem portion which extend rearwardly from the rearmost star. The device in this form does tend to be expelled after a period of time which, although sufficient for promoting growth, is insufficient to enable long term temperature readings to be taken. It has been found, surprisingly, that if the projecting stem and cone portion is removed, leaving only the stars and the connecting portion of the stem therebetween, then virtually 100% retention over extended periods of time (50 days or more) can be achieved.

The length of the arms 16 in the abovementioned prior art unit measured between opposite ends thereof seems to average somewhere slightly in excess of 6 cm. The Applicant has found that for smaller mammalian females like, for example, sows and ewes, a further modified unit having stars with a radius of only about 3.5 cm proved quite adequate although it is preferred that the radius should be at least 3.5 cm, the overall axial length of the device being about 4 cm. As a matter of fact, the full size 2-star modified unit apparently was too large to be inserted all the way to the entrance of the cervix since it remained clearly visible at the vulva opening.

Accordingly, the Applicant has modified this commercially available growth promotion device so as to convert the latter to anchoring device capable of holding small objects in situ within the vagina of mammalian females of various species and sizes for prolonged period of time while, at the same time leaving their normal reproductive functions unimpaired.

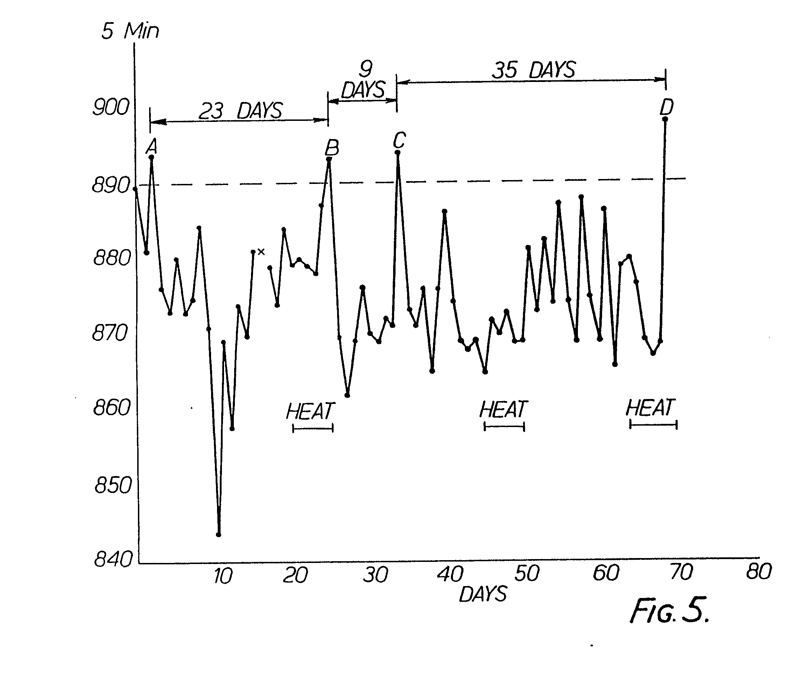

Once the temprature sensing probe is in place as shown, the herd manager or other investigator can begin gathering data for the purpose of ascertaining when the animal is in condition to conceive or, alternatively, is not ovulating and cannot be impregnated. This same data will be effective to indicate the onset of a febrile illness well before any overt clinical signs becomes apparent. The results of actual deep body temperature measurements in three species of female livestock mammals form the subject matter of the graphs appearing herein as Figures 4—9, inclusive, to which detailed reference will soon be made; however, before doing so, it is appropriate to explain more about when the probe should be implanted and why it should be kept in place for an extended period of time.

Cows and other female animals are known to have cyclic variations in basal body temperature which cycle bears a relationship to the estrus cycle. This cycle was discovered to differ a great deal from that of the human female. One major difference is the fact that women experience a near constant temperature pattern from period-to-period, whereas, cattle and other animals do not. The fact of the matter is that farm animals exhibit a changing temperature pattern which seems to depend on some degree at least upon the climate and the season. For instance, it can be demonstrated that the normal body temperature baseline takes on an ascending pattern during prolonged periods of cold weather and a descending one when it is warm. It is essential, therefore, that a fairly long term history of not less than a complete estrus cycle and preferably even longer, say 50 days, be kept and used as the basis for detecting any significant changes such as those that signal ovulation or the onset of febrile illness.

In the human female, despite the constant temperature pattern between periods, ovulation based upon deviations from this pattern cannot be realiably detected. Nonetheless, and contrary to what one might expect, readily detectable temperature spikes signalling ovulation do occur in farm animals. Even though the baseline temperature varies seasonably and with environment conditions, it has been discovered that ovulation can be reliably ascertained provided a sufficient temperature history leading up to the anomaly or spike is available. The proof is, of course, that animals bred on such a spike get pregnant while those bred at other times do not.

More specifically, the estrus cycle is such that a pronounced spike in the order of 0.8°C is noted on the day of estrus in a cow, for example, while an equally prominant dip in temperature takes place on the preceding day and again on the following day when ovulation occurs. This cyclic pattern happens in cows with so-called "silent heats" as well as those with normal heat periods. This 0.8° spike lasts for one day only and it is detected by measuring it against her average body temperature over the preceding ten day period or thereabouts.

In a dairy herd, for example, a cow's greatest milk is produced provided she is inseminated within 90 days after parturition; yet, statistics show that about one-third of all dairy cows miss this target for the reason that over 40% of these cows never have a heat period recorded within the first sixty days after they have calved and an additional 12% or so go over ninety days. Even after the first heat period following parturition is recorded, about one in every six thereafter is missed. For these reasons alone, it is of the utmost importance if cost-effective dairy herd management is to be achieved, that each of these heat periods, and preferably the first, is reliably detected.

Turning the attention next to the graph of Figure 4, wherein Mare I showed four spikes (A, B, C and D) that surpassed a threshold line (spike indicates about 3/4°C above mean). These spikes are spaced at regular intervals that coincide with the expected time between ovulations. Also, spikes A, B and D were associated with estrus. The mare was not teased during the period of spike C, so that estrus status is now known. Each data point (dot) represents a once-daily reading taken at approximately 7.30 a.m. between May 9 and August 12, 1979. The ordinate scale is in radio counts per 5 minute period.

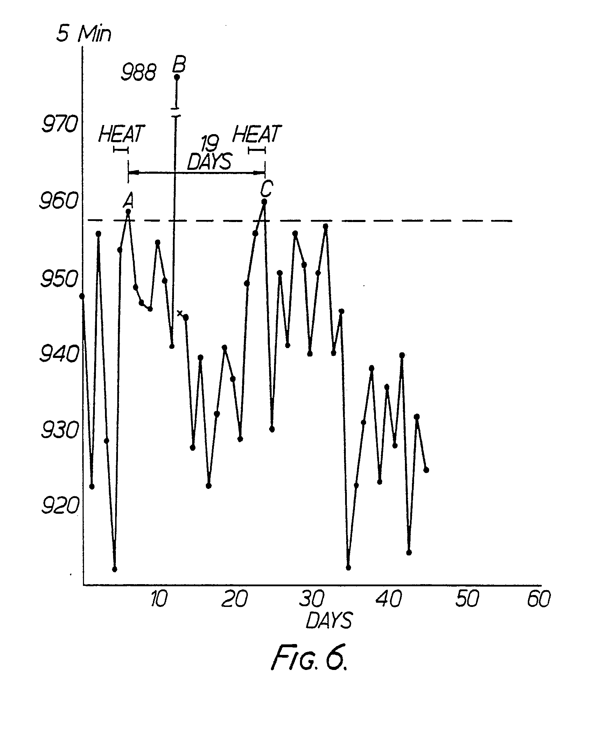

Mare II charted in Figure 5 showed four substantial temperature spikes (magnitude about 3/4°C above mean) which extend above the threshold line. Spikes B and D occurred at the last day of the estrus period. The mare was not teased during the period of spike A, so her receptivity is not known. The spike C occurred during mid-estrus and, curiously, no spike appeared during the subsequent estrus period.

Each data point (dot) represents a once-daily reading taken at approximately 7.30 a.m. between June 1st and August 10, 1979. The left side scale is in radio counts per 5 minute period.

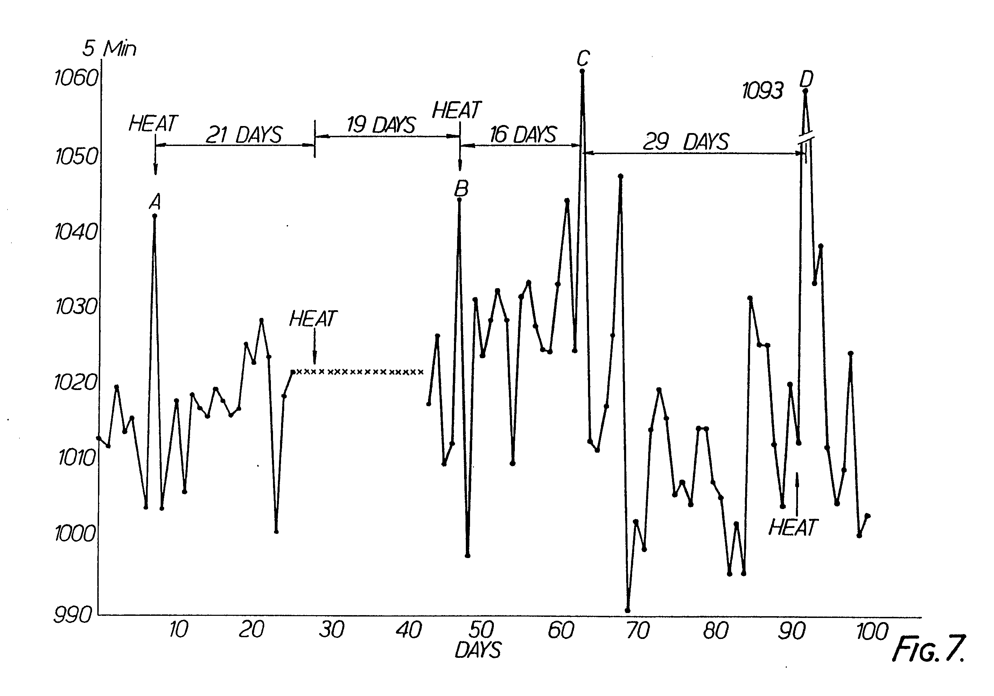

The intravaginal temperature graph of Figure 6 was taken of a sow instead of a mare. The sow exhibited two heat periods of 2 days duration which is characteristic of sows. There were temperature spikes (A and C) towards the end of each heat period. The sow was accidentally bred on the second day after spike C and became pregnant. Spikes A and C were nineteen days apart which matches exactly the normal ovulation interval for sows. Spike B was quite high and indicates a fever of short duration which might well have been due to a mold infection such as a virus might cause.

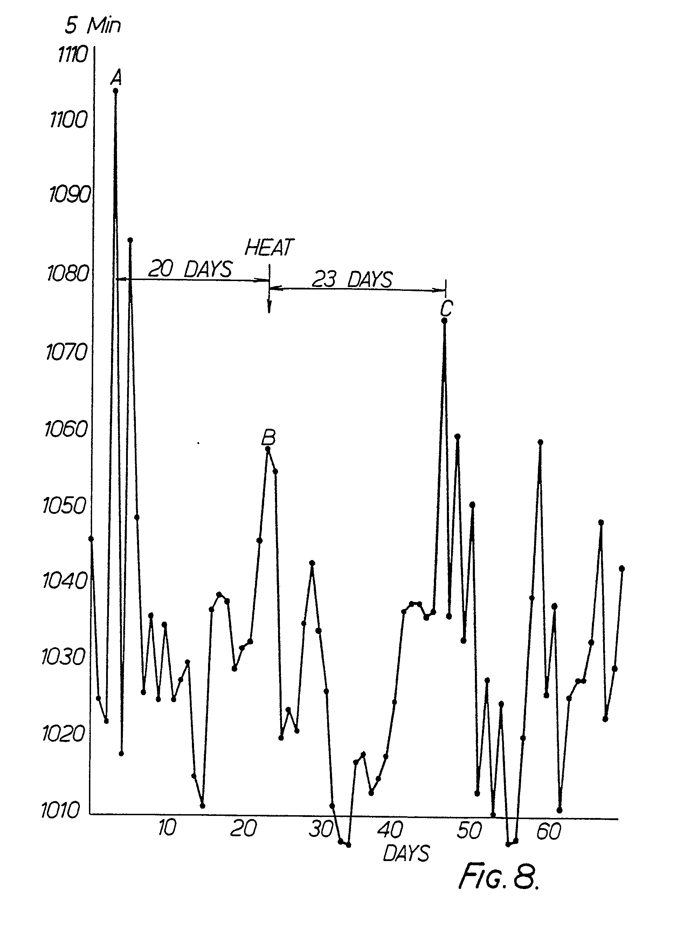

Directing the attention next to Figure 7, heifer 1474 initially experienced three normal heat periods and there were temperature spikes (A and B) recorded as shown. The transmitter was not implanted in the heifer during the period marked xxx. Spike C was not accompanied by standing heat, however, the interval was normal to the previous spike and the heifer was bred. It is believed that she became pregnant and then miscarried because she came in heat 28 days later with a spike occurring the subsequent morning. The 29 day interval was too long for normalcy. Eventually, the heifer was bred again during a heat and spike episode and she became pregnant.

Figure 8 to which reference will next be made details the temperature pattern of yet another heifer. Heifer 1494 presented spikes A, B and C during her test period. She was just reaching puberty when brought into the experiment. She only expressed heat once and that was accompanied by spike B; nevertheless, she did have normal intervals between the three significant spikes. Following spike C she did not show any mating behaviour or spikes during a period of very hot weather. Eventually, she did come in heat and had a smaller spike. She was bred and became pregnant.

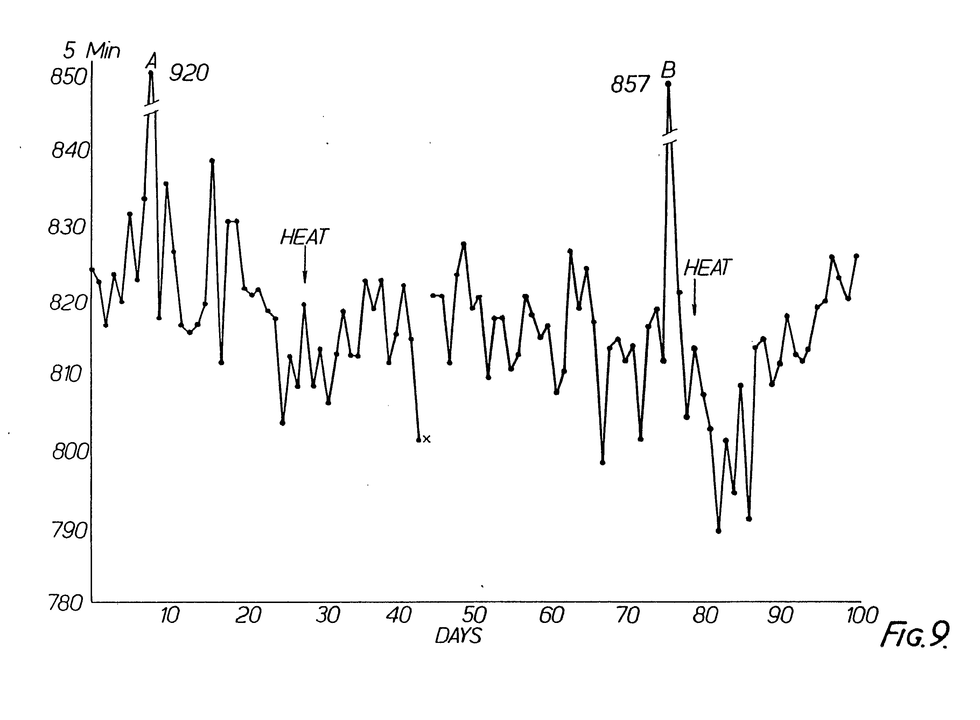

Finally, with reference to Figure 9, a cow 690 was selected because of her infertility as a subject for examination of spike conditions during known reproductive insufficiency. There were two very high spikes without associated heat. She had a quentionable heat once and a definite standing heat towards the end of the observation period. The cow was bred and spike B and did not become pregnant. Apparently, the cow is physiologically out of phase and there was no normal periodicity in her record at all. This example clearly illustrates the value of the remotely-sensed temperature method of the present invention in detecting acyclicity and probably ovulation failure.

On the whole, the foregoing charts clearly reflect the day-to-day physiological conditions of the subjects. When her temperature reading is noticeably greater than her previous ten day average and exceeds all previous highs during that time interval, the probability is that she is preparing to ovulate and she should be bred on either the day of the estrus spike or early the next day. In the specific case of cows, if the foregoing temperature spike falls on a 21 +/— 5 day interval from the preceding spike, the cow is very probably ovulating; however, if the spike is out of phase with the normal estrus cycle as above noted and has a magnitude somewhere around three times the magnitude of previous spikes, the animal is very probably feverish and such a spike signals the onset of some febrile illness rather than estrus and at a time well in advance of when any clinically recognizable symptons appear. The random occurrence of such spikes, their magnitude and duration (more than one day), allow the observer to readily differentiate between the fever spike and the estrus spike. It is also significant to note that the failure to record a spike is equally informative because it signals the absence of ovulation which is every bit as important to know as when the animal is experiencing normal ovulation (see Figure 9).

The foregoing examples clearly demonstrate that, while the temperature cycle of various species of female farm animals have long been recognized as effective indicators of estrus, until now there has never been a reliable, practical and effective method for determining the animal's temperature, deep body or otherwise, on a daily basis under herd management conditions. The method permits the long term monitoring of the deep body temperature of a female mammal without having to handle her over and over again. The subject is natural at all times and need not be agitated as is the case with present deep body temperature measurement methods where the thermometer or other type of temperature measurement probe is repeatedly inserted and removed from her rectum or vulva every single day. The animal is not harmed in any way or otherwise traumatized yet she is constantly providing the observer with much needed informatoin on her physical condition which is otherwise essentially unattainable under field conditions.

Summarizing, the method and device described above solves three heretofore unsolved problems, namely: 1) it provides for remote interrogation and possibly even automated monitoring of an animal's deep body temperature by means of an indwelling probe implanted without surgery; 2) it provides information on ovulation on all animals, both those experiencing active estrus and those who are not; and 3) it detects feverish conditions in advance of clinical illness.

**Claims**

1. A device (24) for intravaginal implantation in a mammalian female comprising an axially extending hub (30) encircled by at least two rings (20A, 20B) of radially-extending springable spine-like fingers (16) co-operating with said hub (30), to define a multi-pointed star, characterised in that a respective said star (20A, 20B) is located at each of the opposed extremities of the hub (30) which is devoid of any projecting portion accessible to the vaginal wall musculature, and in that temperature sensing means (26) are mounted on said hub (30) in nested protective relation among the fingers (16).

2. A device as claimed in claim 1, further characterised in that the temperature sensing means (26) is a telemetric temperature sensory probe (26) which is no longer than the hub (30).

3. A device as claimed in claim 1 or 2, further cahracterised in that the overall axial length thereof measured between the hub ends (18, 18) is substantially less than the diameter of either star (20A, 20B).

4. A device as claimed in any one of claims 1 to 3, further characterised in that at least one additional ring (20C, 20D) of radially-extending springable finges (16) emanates from the hub (30) to define an additional star located intermediate the stars (20A, 20B) on the extremities of the hub (30).

**Patentansprüche**

1. Einrichtung (24) zum intravaginalen Einsetzen in ein weibliches Säugetier, mit einer sich axial erstreckenden Nabe (30), die von mindestens zwei Ringen (20A, 20B) aus sich radial erstreckenden, federfähigen, stachelartigen Fingern (16) umgeben ist, die mit der Nabe (30) zusammenwirken, um einen vielzackigen Stern zu bilden, dadurch gekennzeichnet, daß jeweils ein solcher Stern (20A, 20B) an jedem der entgegengesetzten Enden der Nabe (30) angeordnet ist, welche keinerlei vorspringenden Abschnitt aufweist, der von der Muskulatur der Scheidenwand erreichbar ist, und daß eine Temperaturmeßeinrichtung (26) an der Nabe (30) zwischen den Fingern (16) in geschützter Unterbringung angebracht ist.

2. Einrichtung nach Anspruch 1, dadurch gekennzeichnet, daß die Temperaturmeßeinrichtung (26) eine telemetrische Temperaturmeßsonde (26) ist, die nicht länger ist als die Nabe (30).

3. Einrichtung nach einem der Ansprüche 1 oder 2, dadurch gekennzeichnet, daß ferner die gesamte axiale Länge hiervon, gemessen zwischen den Nabenenden (18, 18), wesentlich kleiner ist als der Durchmesser eines jeden der Sterne (20A, 20B).

4. Einrichtung nach jedem beliebigen der Ansprüche 1 bis 3, ferner dadurch gekennzeichnet, daß mindestens ein zusätzlicher Ring (20C, 20D) aus sich radial erstreckenden, federfähigen Fingern (16) von der Nabe (30) ausgeht, um einen zusätzlichen Stern zu bilden, der zwischen den Sternen (20A, 20B) an den Enden der Nabe (30) angeordnet ist.

**Revendications**

1. Dispositif (24) destiné à être implanté dans le vagin d'une femelle mammifère, comprenant un noyau (30) orienté axialement et entouré par au moins deux couronnes (20A, 20B) de doigts élastiques (16) orientés radialement et semblables à des épines, qui coopèrent avec le noyau (30) pour définir une étoile à multiples branches, caractérisé en ce qu'une telle étoile (20A, 20B) est située à chacune des extrémités opposés du noyau ou tige (30), lequel est dépourvu de toute portion saillante susceptible de venir en contact avec la musculature de la paroi vaginale, et qu'un dispositif (26) pour capter la température est monté sur le noyau (30), entre les doigts (16) et en étant protégé par ceux-ci.

2. Dispositif selon la revendication 1, caractérisé en ce que le dispositif (26) pour capter la température est un capteur de température télémétrique (26) qui n'est pas plus long que le noyau (30).

3. Dispositif selon la revendication 1, caractérisé en ce que sa longueur axiale totale, mesurée entre les extrémités (18) du noyau (30), est sensiblement inférieure au diamètre de l'une ou l'autre étoile (20A, 20B).

4. Dispositif selon l'une quelconque des revendications 1 à 3, caractérisé en ce qu'il comprend au moins une couronne supplémentaire (20C, 20D) de doigts élastiques (16) orientés radialement vers l'extérieur à partir du noyau (30) pour définir une étoile supplémentaire située entre les étoiles (20A, 20B) aux extrémités du noyau (30).

FIG.I.

0 040 077

FIG. 2.

FIG. 3.

FIG. 4.

0 040 077

Fig. 5.

HEAT HEAT HEAT

35 DAYS  9 DAYS  23 DAYS

DAYS

A B C D

5 Min

900 890 880 870 860 850 840

10 20 30 40 50 60 70 80

4

FIG. 6.

FIG. 7.

0 040 077

0 040 077

Fig. 8.

7

Fig.9.

0 040 077